# EUROPEAN PATENT APPLICATION

(11) **EP 4 516 741 A1**
(43) Date of publication of application: **05.03.2025**
(21) Application number: 23796358.2
(22) Date of filing: 25.04.2023
(51) Int. Cl.: C01G 9/02, A61Q 17/04, A61K 8/04, A61K 8/27, C09K 23/54

(54) **SURFACE-TREATED ZINC OXIDE PARTICLES, DISPERSION LIQUID, COSMETIC MATERIAL, METHOD FOR PRODUCING SURFACE-TREATED ZINC OXIDE PARTICLES**

(30) Priority: 27.04.2022 JP 2022073189
(71) Applicant: Sumitomo Osaka Cement Co., Ltd., Tokyo, 105-8641 (JP)
(72) Inventor: MATSUSHITA, Hirokazu, Tokyo 105-8641 (JP); ITO, Tomomi, Tokyo 105-8641 (JP)
(74) Representative: Becker, Eberhard
(86) International application number: PCT/JP2023/016217
(87) International publication number: WO 2023/210618

(57) **Abstract**

The present invention pertains to zinc oxide particles that are surface-treated by using a surface treatment agent. The BET specific surface area of the zinc oxide particles is 1.5-8 m²/g<sp />. The surface treatment agent is an alkyl alkoxysilane having an alkyl group having 6-10 carbon atoms. The contained amount of the surface treatment agent is 0.70-0.92 mass%. The particle diameter D98, which is determined at a cumulative volume percentage of 98% in a dry particle size distribution, is 40 um or less.

## Description

### Technical Field

The present invention relates to surface-treated zinc oxide particles, a dispersion liquid, a cosmetic material, and a method for producing surface-treated zinc oxide particles.

This application claims priority based on Japanese Patent Application No. 2022-073189 filed on April 27, 2022, the content of which is incorporated herein by reference.

### Background Art

Zinc oxide particles having ultraviolet shielding properties have been used in cosmetic materials such as sunscreen and foundation.

In a case where the zinc oxide particles are applied to the cosmetic material, a surface treatment is performed on the zinc oxide particles in order to adapt the surfaces of the zinc oxide particles to properties of cosmetic products or to suppress catalytic activity of the zinc oxide particles. As a surface treatment agent for the zinc oxide particles, for example, metal soaps such as magnesium stearate, silicone oils such as dimethicone and hydrogen dimethicone, and alkylalkoxysilanes such as octyltriethoxysilane (for example, see Patent Literatures 1, 2, and 3).

Among these, the zinc oxide particles surface-treated with alkylalkoxysilane described above exhibit high stability because the alkylalkoxysilane is chemically bonded to the surfaces of the zinc oxide particles.

Furthermore, in the zinc oxide particles as described above, the properties of the particle surface can be easily modified by using a surface treatment agent with different substituents.

The zinc oxide particles surface-treated with alkylalkoxysilane (hereinafter, may be abbreviated as "surface-treated zinc oxide particles") are directly blended into the cosmetic material, or are blended into the cosmetic material in a form of a dispersion liquid obtained by dispersing the zinc oxide particles in a dispersion medium.

### Citation List

### Patent Literature

Patent Literature 1: Japanese Laid-open Patent Publication No. 2002-362925
Patent Literature 2: Japanese Laid-open Patent Publication No. 2001-181136
Patent Literature 3: Japanese Laid-open Patent Publication No. H08-104606

### Summary of Invention

### Problem to Be Solved by Invention

However, the surface-treated zinc oxide particles have a problem of agglomerating with each other during the surface treatment process. The agglomerated surface-treated zinc oxide particles have a significant roughness, resulting in a problem that an excellent feeling of use cannot be achieved when blended in the cosmetic material.

The present invention has been made in view of the above circumstances, and an object of the present invention is to provide surface-treated zinc oxide particles in which feeling of roughness is suppressed. In addition, an object of the present invention is to provide a dispersion liquid and a cosmetic material, containing such surface-treated zinc oxide particles. In addition, an object of the present invention is to provide a method for producing such surface-treated zinc oxide particles.

### Means for Solving Problem

In order to achieve the above-described objects, surface-treated zinc oxide particles according to a first aspect of the present invention are zinc oxide particles surface-treated with a surface treatment agent, in which a BET specific surface area of the zinc oxide particles is 1.5 m²/g or higher and 8 m²/g or lower, the surface treatment agent is an alkylalkoxysilane having an alkyl group having 6 or more and 10 or less carbon atoms, a content of the surface treatment agent is 0.70% by mass or more and 0.92% by mass or less, and a particle diameter D98 in a case where a cumulative volume percentage in a dry particle size distribution is 98% is 40 µm or lower.

In the surface-treated zinc oxide particles according to the first aspect of the present invention, the surface treatment agent may be at least one of octyltrimethoxysilane or octyltriethoxysilane.

A dispersion liquid according to a second aspect of the present invention contains the above-described surface-treated zinc oxide particles and a dispersion medium.

A cosmetic material according to a third aspect of the present invention contains at least one selected from the group consisting of the above-described surface-treated zinc oxide particles and the above-described dispersion liquid.

A method for producing surface-treated zinc oxide particles according to a fourth aspect of the present invention is a method for producing the above-described surface-treated zinc oxide particles, the method including: a step of subjecting zinc oxide particles to a surface treatment by mixing the zinc oxide particles with a surface treatment agent; a step of subjecting the surface-treated zinc oxide particles to a heat treatment; and a step of cracking the surface-treated zinc oxide particles after the heat treatment, in which a BET specific surface area of the zinc oxide particles is 1.5 m²/g or higher and 8 m²/g or lower, the surface treatment agent is an alkylalkoxysilane having an alkyl group having 6 or more and 10 or less carbon atoms, a mixing amount of the surface treatment agent is 1.2 parts by mass or more and 3.5 parts by mass or less with respect to 100 parts by mass of the zinc oxide particles, and in the step of subjecting the zinc oxide particles to the surface treatment, a content of a solvent is 2% by mass or less with respect to a total mass of the zinc oxide particles and the surface treatment agent.

### Advantageous Effects of Invention

According to the present invention, it is possible to provide surface-treated zinc oxide particles in which feeling of roughness is suppressed. In addition, according to the present invention, it is possible to provide a dispersion liquid and a cosmetic material, containing such surface-treated zinc oxide particles. In addition, according to the present invention, it is possible to provide a method for producing such surface-treated zinc oxide particles.

### Description of Embodiments

Examples of preferred embodiments of the surface-treated zinc oxide particles, the dispersion liquid, the cosmetic material, and the method for producing surface-treated zinc oxide particles according to the present invention will be described below.

The present embodiments are simply specific descriptions for better understanding of the gist of the invention and do not limit the present invention unless particularly otherwise described. For example, unless otherwise specified, materials, amounts, types, numbers, sizes, ratios, conditions such as temperatures, and the like may be changed, added, or omitted as necessary. In the embodiments described below, preferred examples from each embodiment may be exchanged or shared.

### [Surface-treated zinc oxide particles]

The surface-treated zinc oxide particles according to the present embodiment are zinc oxide particles surface-treated with a surface treatment agent, in which a BET specific surface area of the zinc oxide particles is 1.5 m²/g or higher and 8 m²/g or lower, the surface treatment agent is an alkylalkoxysilane having an alkyl group having 6 or more and 10 or less carbon atoms, a content of the surface treatment agent is 0.70% by mass or more and 0.92% by mass or less, and a particle diameter D98 in a case where a cumulative volume percentage in a dry particle size distribution (hereinafter, may be abbreviated as "D98") is 98% is 40 µm or lower. The surface treatment of the surface-treated zinc oxide particles according to the present embodiment refers to modifying surfaces of the zinc oxide particles with the surface treatment agent. That is, the surface treatment of the surface-treated zinc oxide particles according to the present embodiment refers to forming a coat consisting of the surface treatment agent on at least a part of the surfaces of the zinc oxide particles, or adhering the surface treatment agent to at least a part of the surfaces of the zinc oxide particles.

In the surface-treated zinc oxide particles according to the present embodiment, the zinc oxide particles having a specific BET specific surface area are surface-treated with a specific alkylalkoxysilane at a specific mass ratio, without adding a solvent. Therefore, agglomeration between the zinc oxide particles and agglomeration between the surface-treated zinc oxide particles are suppressed, and surface-treated zinc oxide particles having a small D98 can be obtained. Since the surface-treated zinc oxide particles in which the agglomeration is suppressed as described above have a reduced feeling of roughness when applied to the skin, the surface-treated zinc oxide particles are suitably used in a cosmetic material.

In the surface-treated zinc oxide particles according to the present embodiment, a content of Si is preferably 0.06% by mass or more and 0.30% by mass or less. The content of Si may be 0.07% by mass or more and 0.25% by mass or less, 0.08% by mass or more and 0.20% by mass or less, 0.09% by mass or more and 0.15% by mass or less, 0.10% by mass or more and 0.13% by mass or less, or the like. In a case where the content of Si is within the above-described range, the surfaces of the above-described zinc oxide particles are sufficiently hydrophobized. As a result, in a case where the surface-treated zinc oxide particles according to the present embodiment are blended into a cosmetic material, the feeling of use is excellent.

In the surface-treated zinc oxide particles according to the present embodiment, a hydroxyl group treatment rate is preferably 98% by mass or more. In a case where the hydroxyl group treatment rate is 98% by mass or more, the agglomeration between the surface-treated zinc oxide particles is suppressed, and surface-treated zinc oxide particles having a small D98 can be obtained.

In the present specification, the "BET specific surface area" means a value measured by a BET method using a specific surface area measuring device, such as a fully automatic specific surface area measuring device (trade name: Macsorb HM Model-1201, manufactured by MOUNTECH Co., Ltd.) .

In the present specification, the "content of the surface treatment agent" is calculated using the following general formula (1). Content of surface treatment agent = (Ignition loss - Drying loss) × Molecular weight of surface treatment agent/Molecular weight of alkyl group in surface treatment agent

In the present specification, the "drying loss" means the loss in mass after drying at 105°C for 2 hours, which can be determined by the following method.

First, 2 g of the surface-treated zinc oxide particles is prepared. The surface-treated zinc oxide particles are preferably particles stored under dry conditions. The particles are heated in a dryer set at 105°C for 2 hours, and mass after the heating is measured and a mass reduction rate is regarded as the drying loss (% by mass).

That is, the drying loss can be obtained using the above-described measurement results from the following formula (2). Drying loss of surface-treated zinc oxide particles (% by mass) = (Mass of surface-treated zinc oxide particles before heating - Mass of surface-treated zinc oxide particles after heating)/Mass of surface-treated zinc oxide particles before heating × 100

In the present specification, the "ignition loss" means the loss in mass after heating at 500°C for 4 hours, which can be determined by the following method.

First, 2 g of the surface-treated zinc oxide particles is prepared. The surface-treated zinc oxide particles are preferably particles stored under dry conditions. The particles are heated in a dryer set at 500°C for 4 hours, and mass after the heating is measured and a mass reduction rate is regarded as the ignition loss (% by mass).

That is, the ignition loss can be obtained using the above-described measurement results from the following formula (3). Ignition loss of surface-treated zinc oxide particles (% by mass) = (Mass of surface-treated zinc oxide particles before heating - Mass of surface-treated zinc oxide particles after heating)/Mass of surface-treated zinc oxide particles before heating × 100

In a case where octyltrimethoxysilane is used as the surface treatment agent, a molecular weight of the surface treatment agent, that is, the molecular weight of octyltriethoxysilane is 276.49. The molecular weight of alkyl groups in the octyltriethoxysilane is 113.08. The content of the surface treatment agent can be calculated using the ratio of the molecular weights with reference to the formula (1).

The drying loss can be considered as a mass reduction due to removal of moisture contained in the surface-treated zinc oxide particles and impurities other than the surface-treated zinc oxide particles, such as unreacted surface treatment agent, by heating at 105°C.

The ignition loss can be considered as a mass reduction due to the removal of the above-described impurities and removal of the alkyl group from the alkylalkoxysilane adhering to the zinc oxide particles by heating at 500°C.

Therefore, in the present embodiment, a content of the surface treatment agent actually adhering to the zinc oxide particles by the surface treatment is defined as the content of the surface treatment agent calculated by the above-described general formula (1).

In the present specification, the "content of Si" means a value measured by the following method using a spectroscopic analysis apparatus, such as an ICP emission spectrometry apparatus ICP-AES 700-ES (manufactured by Varian, Inc.).

0.2 g of the surface-treated zinc oxide particles to be measured is placed into a platinum crucible, and the temperature is gradually raised to 700°C in an electric furnace to ash the surface-treated zinc oxide particles. 2 g of lithium tetraborate is added to the ashed sample, and the sample is heated in the electric furnace to 925°C to be molten. Next, the molten sample is placed into a 100 mL tall beaker along with the platinum crucible. Next, 70 mL of warm water and 8 mL of nitric acid are added to the tall beaker, and the mixture is heated and stirred with a hot stirrer to dissolve the sample. The dissolved solution is transferred to a 200 mL volumetric flask and brought to volume, and this solution is used as a sample solution. The sample solution may be diluted as needed. A Y standard solution is added to the sample solution so that the concentration of Y is to be 1,000 ppm. A calibration curve is created using an element standard solution with a known concentration. Y as an internal standard substance, the lithium tetraborate, and the nitric acid are added to the element standard solution for creating the calibration curve, such that the concentrations thereof are the same as those of the sample solution. The sample solution is measured using the ICP emission spectrometry apparatus, and quantified by a calibration curve method.

Since the surface treatment agent used in the present embodiment is alkylalkoxysilane, the content of the surface treatment agent adhering to the zinc oxide particles can be measured based on the content of Si.

In the present specification, the "D98" means a value in a case where a volume-based particle size distribution is measured in a dry manner using a particle size distribution measuring device, such as a laser diffraction-type particle size distribution measuring device (model: Mastersizer 3000, manufactured by Malvern Panalytical Ltd.), and a cumulative volume percentage thereof is 98%.

In the present specification, the "hydroxyl group treatment rate" is measured using a red coloring agent which absorbs light at a wavelength of around 545 nm, as represented by General Formula (4).

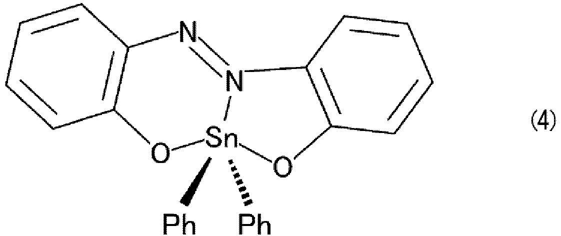

The red coloring agent represented by General Formula (4) can be produced by the following method.

1 mmol of 2,2'-dihydroxyazobenzene, 1 mmol of diphenyltin(IV) oxide as a metal source, and 30 mL of acetone are mixed with each other to prepare a mixed solution.

Next, the mixed solution is stirred at 70°C for 3 hours to perform a dehydration reaction, coordinating the diphenyltin oxide with the 2,2'-dihydroxyazobenzene.

The mixed solution after the dehydration reaction is filtered, the filtrate is collected, and the solvent is distilled off from the filtrate to obtain the red coloring agent represented by General Formula (4).

The above-described red coloring agent represented by General Formula (4) is selectively adsorbed onto hydroxyl groups present on the surfaces of the zinc oxide particles, and does not react with hydroxyl groups in substances such as water or alcohol. Therefore, the amount of metal hydroxyl groups contained in the zinc oxide particles or the surface-treated zinc oxide particles can be qualitatively and quantitatively evaluated without being affected by moisture. That is, by examining the amount of the above-described red coloring agent adsorbed on the zinc oxide particles before the surface treatment and the amount of the above-described red coloring agent adsorbed on the surface-treated zinc oxide particles, hydrophobicity of the surfaces of the zinc oxide particles can be determined. That is, as a treatment rate of the hydroxyl groups on the surfaces of the zinc oxide particles is higher, the hydroxyl groups present on the surfaces of the zinc oxide particles are more surface-treated and hydrophobized.

Specifically, the hydroxyl group treatment rate (%) with the above-described red coloring agent can be measured by the following method.

250 nmol (0.12 mg) of the red coloring agent represented by General Formula (4) is dissolved in toluene to prepare 5 mL of a solution C1 for evaluation, having a concentration of 5 × 10⁻⁵ mol/L. An absorbance C2 of the solution C1 at a wavelength of 545 nm is measured.

x g of the zinc oxide particles before the surface treatment is added to the above-described solution C1 for evaluation, and the mixture is stirred and mixed at 60°C for 4 hours to prepare a mixed solution. x is, for example, approximately 4 × 10⁻³ g. The zinc oxide particles are removed from the mixed solution by centrifugal separation to obtain a mixed solution A1 for evaluation. An absorbance A2 of the mixed solution A1 at a wavelength of 545 nm is measured.

y g of the surface-treated zinc oxide particles to be measured is added to the above-described solution C1 for evaluation, and the mixture is stirred and mixed at 60°C for 4 hours to prepare a mixed solution. y is approximately 4 × 10⁻³ g. The surface-treated zinc oxide particles are removed from the mixed solution by centrifugal separation to obtain a mixed solution B1 for evaluation. An absorbance B2 of the mixed solution B1 at a wavelength of 545 nm is measured.

From the following general formula (5), an amount (mol/m²) of the above-described red coloring agent adsorbed on the zinc oxide particles before the surface treatment is calculated. Adsorption amount A3 = ((A2 - C2)/C2) × 250 × 10-9 (mol)/x (g)

From the following general formula (6), an amount (mol/m²) of the red coloring agent adsorbed on the surface-treated zinc oxide particles is calculated. Adsorption amount B3 = ((B2 - C2)/C2) × 250 × 10-9 (mol) /y (g)

In the general formulae (5) and (6), since a decrease in absorbance indicates that the coloring agent is adsorbed, the adsorption amount of the above-described red coloring agent is calculated based on an idea that the decrease rate in absorbance can be converted to the adsorption rate of the coloring agent.

The treatment rate of the hydroxyl groups can be calculated by the following general formula (7). Treatment rate of hydroxyl groups (%) = 100 - ((B3/A3) × 100)

### (BET specific surface area of surface-treated zinc oxide particles)

A BET specific surface area of the surface-treated zinc oxide particles can be optionally selected, but is preferably 1.5 m²/g or higher, more preferably 2.5 m²/g or higher, still more preferably 3.0 m²/g or higher, and particularly preferably 3.5 m²/g or higher. In addition, the BET specific surface area of the surface-treated zinc oxide particles is preferably 8 m²/g or lower, preferably 8.0 m²/g or lower, more preferably 7.5 m²/g or lower, and still more preferably 7 m²/g or lower. As necessary, the BET specific surface area of the surface-treated zinc oxide particles may be 6.5 m²/g or lower, or 6.0 m²/g or lower. The upper limit value and lower limit value of the BET specific surface area of the surface-treated zinc oxide particles can be combined randomly.

In a case where the BET specific surface area of the surface-treated zinc oxide particles is 1.5 m²/g or higher and 8 m²/g or lower, the transparency and ultraviolet shielding properties are excellent when blended into a cosmetic material.

A value of the BET specific surface area of the zinc oxide particles before the surface treatment and the value of the BET specific surface area of the surface-treated zinc oxide particles after the surface treatment do not significantly change.

### (Average primary particle diameter of surface-treated zinc oxide particles)

An average primary particle diameter of the surface-treated zinc oxide particles according to the present embodiment is preferably 130 nm or higher, more preferably 150 nm or higher, and still more preferably 200 nm or higher. In addition, the average primary particle diameter of the surface-treated zinc oxide particles according to the present embodiment is preferably 300 nm or lower, more preferably 270 nm or lower, and still more preferably 250 nm or lower.

In a case where the average primary particle diameter of the surface-treated zinc oxide particles is 130 nm or higher and 300 nm or lower, the transparency and ultraviolet shielding properties are excellent when blended into a cosmetic material.

The average primary particle diameter of the surface-treated zinc oxide particles described above can be calculated using the following general formula (8) with the BET specific surface area of the surface-treated zinc oxide particles described above. Average primary particle diameter (nm) = 6000/(BET specific surface area (m2/g) × ρ (g/cm3)) (in the formula, ρ is a density of the zinc oxide particles, which is 5.61 g/cm³)

Alternatively, the average primary particle diameter of the surface-treated zinc oxide particles described above may be determined by the following method. That is, in a case where the above-described surface-treated zinc oxide particles are observed using a transmission electron microscope (TEM) or the like, a predetermined number of the surface-treated zinc oxide particles, for example, 200 or 100, are selected. Next, the longest linear portion (maximum long diameter) of each of the surface-treated zinc oxide particles is measured, and these measured values are arithmetically averaged.

In a case where the surface-treated zinc oxide particles are agglomerated, an agglomerated particle diameter of agglomerate is not measured. The predetermined number of the surface-treated zinc oxide particles (primary particles) constituting the agglomerate is measured, and the average primary particle diameter is defined based on these measurements.

### (Zinc oxide particles)

The BET specific surface area of the zinc oxide particles according to the present embodiment is 1.5 m²/g or higher and 8 m²/g or lower, preferably 2.5 m²/g or higher and 7.0 m²/g or lower. The BET specific surface area of the zinc oxide particles may be 3.5 m²/g or higher and 6.5 m²/g or lower, or 4.5 m²/g or higher and 6.0 m²/g or lower. In a case where the BET specific surface area of the zinc oxide particles is lower than the above-described lower limit value, the transparency decreases when blended in a cosmetic material, which is not preferable. In a case where the BET specific surface area of the zinc oxide particles is higher than the above-described upper limit value, the particles may easily agglomerate when the surface-treated zinc oxide particles are contained in the cosmetic material at a high concentration, which is not preferable.

The BET specific surface area of the zinc oxide particles according to the present embodiment means a value measured by a BET method using a specific surface area measuring device, such as a fully automatic specific surface area measuring device (trade name: Macsorb HM Model-1201, manufactured by MOUNTECH Co., Ltd.), as described above.

An average primary particle diameter of the zinc oxide particles according to the present embodiment is preferably 130 nm or higher, more preferably 150 nm or higher, and still more preferably 200 nm or higher. In addition, the average primary particle diameter of the zinc oxide particles according to the present embodiment is preferably 300 nm or lower, more preferably 270 nm or lower, and still more preferably 250 nm or lower.

In a case where the average primary particle diameter of the above-described zinc oxide particles is 130 nm or higher and 300 nm or lower, the transparency and ultraviolet shielding properties are excellent when blended into a cosmetic material.

The average primary particle diameter of the zinc oxide particles described above can be calculated using the general formula (8) formula described above, based on the BET specific surface area of the zinc oxide particles described above, in the same manner as the BET-converted particle diameter of the surface-treated zinc oxide particles described above.

In addition, the average primary particle diameter of the zinc oxide particles described above may be measured using the transmission electron microscope, in the same manner as the average primary particle diameter of the surface-treated zinc oxide particles described above.

In the present embodiment, by performing the surface treatment on the zinc oxide particles, the BET specific surface area of the surface-treated zinc oxide particles tends to be smaller than the BET specific surface area of the zinc oxide particles before the surface treatment. However, the BET specific surface area of the surface-treated zinc oxide particles and the BET specific surface area of the zinc oxide particles before the surface treatment are substantially the same. Similarly, by performing the surface treatment on the zinc oxide particles, the average primary particle diameter of the surface-treated zinc oxide particles tends to be larger than the average primary particle diameter of the zinc oxide particles before the surface treatment. However, the average primary particle diameter of the surface-treated zinc oxide particles and the average primary particle diameter of the zinc oxide particles before the surface treatment are substantially the same. Here, the "substantially the same" means that a difference in BET specific surface area between the zinc oxide particles and the surface-treated zinc oxide particles is approximately 5 m²/g or lower.

In the present embodiment, from the viewpoint of improving dispersion stability in the cosmetic material, it is preferable to use high-purity zinc oxide particles.

### (Surface treatment agent)

The surface treatment agent according to the present embodiment is an alkylalkoxysilane having an alkyl group having 6 or more and 10 or less carbon atoms, and a silane coupling agent represented by General Formula (9), which can be used as a silane coupling agent suitable for use in cosmetic materials, can be preferably used.

R¹ₙSi(OR²)₄₋ₙ ... (9)

(here, R¹ represents an alkyl group having 6 to 10 carbon atoms, R² represents an alkyl group having 1 to 4 carbon atoms, and n represents 1 to 3)
R¹ is preferably an alkyl group having 7 to 9 carbon atoms, and more preferably an alkyl group having 8 carbon atoms.
R² is preferably an alkyl group having 1 or more and 3 or less carbon atoms, and more preferably an alkyl group having 1 or more and 2 or less carbon atoms.
n is preferably 1 or more and 2 or less, and more preferably 1.

Specifically, examples of the surface treatment agent according to the present embodiment include alkylalkoxysilanes such as hexyltrimethoxysilane, hexyltriethoxysilane, heptyltrimethoxysilane, heptyltriethoxysilane, octyltrimethoxysilane, octyltriethoxysilane (triethoxycaprylylsilane), nonyltrimethoxysilane, nonyltriethoxysilane, decyltrimethoxysilane, and decyltriethoxysilane. Among these, alkylalkoxysilane having an octyl group in the molecule is preferable.

Specifically, octyltriethoxysilane or octyltrimethoxysilane is preferable due to moderate polarity of the functional group, which allows it to be compatible with a wide range of oil phases, from natural oils and ester oils to silicone oils; and octyltriethoxysilane is particularly preferable.

The surface treatment using the above-described surface treatment agent is preferably performed by mixing the zinc oxide particles with the surface treatment agent in an amount of 1.2 parts by mass or more and 3.5 parts by mass or less with respect to 100 parts by mass of the zinc oxide particles. The above-described amount may be 1.5 parts by mass or more and 3.3 parts by mass or less, 1.8 parts by mass or more and 3.0 parts by mass or less, or 2.0 parts by mass or more and 2.8 parts by mass or less.

In a case where the zinc oxide particles are surface-treated with the surface treatment agent by a method described later in an amount of 1.2 parts by mass or more and 3.5 parts by mass or less with respect to 100 parts by mass of the zinc oxide particles, the content of the surface treatment agent calculated by the general formula (1) described above is 0.70% by mass or more and 0.92% by mass or less. The content of the surface treatment agent is preferably 0.72% by mass or more and 0.90% by mass or less, and more preferably 0.75% by mass or more and 0.85% by mass or less.

In addition, assuming that the total amount of the surface-treated zinc oxide particles is 100% by mass, the content of Si, measured by the above-described ICP emission spectrometry apparatus, is 0.06% by mass or more and 0.30% by mass or less.

In a case where the content of the surface treatment agent contained in the surface-treated zinc oxide particles is within the above-described range, the surfaces of the zinc oxide particles are sufficiently hydrophobized. As a result, in a case where the surface-treated zinc oxide particles according to the present embodiment are blended into a cosmetic material, the feeling of use is excellent. In a case where the content of the surface treatment agent contained in the surface-treated zinc oxide particles is lower than the above-described lower limit value, the surfaces of the zinc oxide particles are not sufficiently hydrophobized, and the zinc oxide particles are easily agglomerate with each other through untreated surfaces, which is not preferable because the D98 is increased. In addition, this is not preferable because texture of the surface-treated zinc oxide particles to be worse. In a case where the content of the surface treatment agent contained in the surface-treated zinc oxide particles is higher than the above-described upper limit value, the excess surface treatment agent causes the surface-treated zinc oxide particles to agglomerate, which is not preferable because the D98 is increased. In addition, this is not preferable because texture of the surface-treated zinc oxide particles to be worse.

In addition, in the surface-treated zinc oxide particles according to the present embodiment, the zinc oxide particles has a BET specific surface area of 1.5 m²/g or higher and 8 m²/g or lower and the content of the surface treatment agent is 0.70% by mass or more and 0.92% by mass or less with respect to 100% by mass of the total mass of the surface-treated zinc oxide particles, and it is important that the amount of the surface treatment agent is small.

By performing, on the zinc oxide particles having a BET specific surface area of 1.5 m²/g or higher and 8 m²/g or lower, the surface treatment with a small amount of the surface treatment agent and without adding a solvent, an increase in b* in a L*a*b* color system chromaticity diagram is suppressed, and surface-treated zinc oxide particles having a D98 of 40 µm or lower can be obtained.

In the surface-treated zinc oxide particles according to the present embodiment, the treatment rate of the hydroxyl groups, calculated by the above-described general formula (7), is preferably 98% or more, more preferably 99% or more, and still more preferably 99.5% or more, using the above-described red coloring agent represented by General Formula (4). For example, the treatment rate of the hydroxyl groups may be 98.5% by mass or more, 99.0% by mass or more, or 99.9% by mass or more.

Since the hydroxyl group treatment rate is within the above-described range, the surfaces of the zinc oxide particles are appropriately surface-treated and hydrophobized, allowing the surface-treated zinc oxide particles to be easily blended with oil-based cosmetic materials.

In the L*a*b* color system chromaticity diagram of the surface-treated zinc oxide particles according to the present embodiment, b* is preferably 6.3 or less.

The lower limit value of b* is preferably 0, but may be 2.0 or more, 3.0 or more, 4.0 or more, or 5.0 or more.

In a case where b* in the L*a*b* color system chromaticity diagram of the surface-treated zinc oxide particles is 6.3 or less, it is possible to suppress unintentional coloring when the surface-treated zinc oxide particles are blended into a cosmetic material.

As a method of measuring b* in the L*a*b* color system chromaticity diagram of the surface-treated zinc oxide particles, a known method such as using a spectrophotometer (manufactured by Tokyo Denshoku CO., LTD., Spectro Color Meter SE7700) can be adopted.

The D98 of the surface-treated zinc oxide particles according to the present embodiment is 40 µm or lower, preferably 38 µm or lower, more preferably 36 µm or lower, and still more preferably 34 µm or lower.

In a case where the D98 is higher than 40 µm, the feeling of roughness of the surface-treated zinc oxide particles is significant, and it is difficult to obtain a cosmetic material having excellent feeling of use, when the surface-treated zinc oxide particles are blended into the cosmetic material.

As long as characteristics of the surface-treated zinc oxide particles according to the present embodiment are not inhibited, in addition to the above-described alkylalkoxysilane, a surface treatment agent used in cosmetic materials, other than the alkylalkoxysilane, may be used to perform the surface treatment on the zinc oxide particles.

As the surface treatment agent other than the above-described alkylalkoxysilane, inorganic materials such as silica and alumina, or organic materials such as a silicone compound, fatty acid, fatty acid soap, fatty acid ester, and organic titanate compound can be used.

It is preferable that the surface-treated zinc oxide particles according to the present embodiment are produced by a method in which the zinc oxide particles with a specific BET specific surface area are treated with the specific surface treatment agent at a specific mass ratio without adding a solvent, in which the D98 is 40 µm or lower. Therefore, in the surface-treated zinc oxide particles according to the present embodiment, the surfaces of the zinc oxide particles are sufficiently hydrophobized, the feeling of roughness is suppressed, the increase in b* is suppressed, and the feeling of use when blended into a cosmetic material is excellent.

### [Method for producing surface-treated zinc oxide particles]

The method for producing surface-treated zinc oxide particles according to the present embodiment includes a step of subjecting zinc oxide particles to a surface treatment by mixing the zinc oxide particles with a surface treatment agent (hereinafter, referred to as "surface treatment step"), a step of subjecting the surface-treated zinc oxide particles to a heat treatment (hereinafter, referred to as "heat treatment step"), and a step of cracking the surface-treated zinc oxide particles after the heat treatment (hereinafter, referred to as "cracking step"). A BET specific surface area of the above-described zinc oxide particles is 1.5 m²/g or higher and 8 m²/g or lower, the above-described surface treatment agent is an alkylalkoxysilane having an alkyl group having 6 or more and 10 or less carbon atoms, and a mixing amount of the above-described surface treatment agent is 1.2 parts by mass or more and 3.5 parts by mass or less with respect to 100 parts by mass of the above-described zinc oxide particles. In the above-described surface treatment step, a content of a solvent is 2% by mass or less with respect to the total mass of the above-described zinc oxide particles and the above-described surface treatment agent.

Here, the fact that the content of the solvent is 2% by mass or less in the surface treatment step means that solvents generated by side reactions, such as absorbed water contained in the zinc oxide particles or alcohol generated by the hydrolysis reaction of the surface treatment agent, may be present, but water or an organic solvent is not added to promote the surface treatment reaction. That is, in the surface treatment step according to the present embodiment, no solvent is added, or if added, the amount of solvent is extremely small. The "content of the solvent is 2% by mass or less" means "addition amount of the solvent is 0% by mass".

The same zinc oxide particles and surface treatment agent as described above can be used.

In order to uniformly perform the surface treatment on the surfaces of the particles, the surface treatment is generally performed by a wet manner. In addition, even in a case where the surface treatment is performed in a dry manner, it is generally common to add a small amount of an organic solvent such as alcohol for the surface treatment.

The present inventors also have performed the surface treatment of the zinc oxide particles in a dry manner by adding solvents such as water and alcohol.

However, the present inventors have noticed that, during the process of removing the solvent, agglomeration of the zinc oxide particles is likely to occur.

Therefore, a surface treatment method without adding water or an organic solvent has been attempted, but without the addition of water or an organic solvent, the surfaces of the zinc oxide particles cannot be sufficiently hydrophobized.

As a result of various trials and errors, the present inventors have found that, by using the zinc oxide particles having a BET specific surface area of 1.5 m²/g or higher and 8 m²/g or lower, and performing the surface treatment with 1.2 parts by mass or more and 3.5 parts by mass or less of the above-described alkylalkoxysilane with respect to 100 parts by mass of the zinc oxide particles, the surface treatment is sufficiently performed without adding an organic solvent such as alcohol or water, and agglomeration of the surface-treated zinc oxide particles is suppressed.

The zinc oxide particles used in cosmetic materials are required to have high transparency, and therefore, zinc oxide particles having a BET specific surface area of 20 m²/g or higher are usually used. However, the present inventors have found that, by using the zinc oxide particles having a BET specific surface area of 1.5 m²/g or higher and 8 m²/g or lower, the agglomeration of the surface-treated zinc oxide particles is suppressed, these surface-treated zinc oxide particles exhibit excellent transparency and ultraviolet shielding properties when blended into a cosmetic material, and the feeling of roughness is suppressed.

During the surface treatment step of the method for producing surface-treated zinc oxide particles according to the present embodiment, it is preferable not to add an organic solvent such as alcohol, and it is more preferable not to add either water or an organic solvent.

Since it is difficult to completely remove moisture absorbed from the air, and the surface treatment reaction does not proceed when the adsorbed water is 0, water is inevitably contained in the zinc oxide particles. In addition, in a case where the hydrolysis reaction of the surface treatment agent proceeds, alcohol derived from the alkoxysilane is generated. However, in a case where water or an organic solvent is added during the surface treatment step, a removal step is necessary, and this removal step causes the surface-treated zinc oxide particles to agglomerate. Therefore, during the surface treatment step of the method for producing surface-treated zinc oxide particles according to the present embodiment, in the surface treatment step, the content of the solvent is set to 2% by mass or less with respect to the total mass of the zinc oxide particles and the surface treatment agent. In a case where the content of the solvent is 2% by mass or less, the agglomeration of the surface-treated zinc oxide particles can be suppressed during the above-described heat treatment step. The addition amount of the organic solvent is 0% by mass, and the addition amount of water and the organic solvent is also 0% by mass. In other words, in all the steps of the method for producing surface-treated zinc oxide particles according to the present embodiment, the addition amount of water and the organic solvent is 0% by mass. That is, the content of solvents other than the absorbed water contained in the zinc oxide particles or the alcohol generated by the hydrolysis reaction of the surface treatment agent is 0% by mass. This means that additives, catalysts, or impurities which are inevitably contained are not removed to the extent that they do not affect the effect of the present invention.

In the above-described surface treatment step, the zinc oxide particles having a BET specific surface area of 1.5 m²/g or higher and 8 m²/g or lower are stirred in a mixer such as a Henschel mixer and a super mixer, while adding the above-described alkylalkoxysilane to the zinc oxide particles in a form of liquid droplets or by spray. Subsequently, the zinc oxide particles and the alkylalkoxysilane are stirred at high speed for a certain period of time.

The stirring may be carried out at room temperature or at an optionally selected temperature, for example, 30°C to 100°C.

A stirring speed is not particularly limited as long as it is a speed at which the zinc oxide particles and the alkylalkoxysilane are mixed and the surface treatment reaction proceeds. For example, the stirring can be carried out at a circumferential speed of 5 m/s to 60 m/s.

A stirring time is not particularly limited as long as it is a time at which the zinc oxide particles and the alkylalkoxysilane are mixed and the surface treatment reaction proceeds. For example, the stirring can be carried out for 1 minute to 1 hour, preferably for approximately 10 minutes to 30 minutes.

The mixing amount of the above-described alkylalkoxysilane is 1.2 parts by mass or more and 3.5 parts by mass or less with respect to 100 parts by mass of the above-described zinc oxide particles. The mixing amount of the above-described alkylalkoxysilane is preferably 1.4 parts by mass or more and 3.0 parts by mass or less, more preferably 1.6 parts by mass or more and 2.5 parts by mass or less, and still more preferably 1.8 parts by mass or more and 2.3 parts by mass or less.

In the above-described heat treatment step, the heat treatment may be appropriately performed at a temperature for a time such that the surface treatment proceeds and the surfaces of the zinc oxide particles are hydrophobized. For example, the heat treatment can be performed at a temperature of 70°C to 200°C for 30 minutes to 24 hours. The atmosphere during the heat treatment is not particularly limited as long as the surface treatment is not inhibited, and the atmosphere may be any of an air atmosphere, an oxygen atmosphere, an inert atmosphere, a reduced pressure atmosphere, or a vacuum atmosphere.

The heat treatment step may be carried out while stirring.

The above-described cracking step is not particularly limited as long as it is a method which can crack the surface-treated zinc oxide particles after the heat treatment such that the D98 of the surface-treated zinc oxide particles is 40 µm or lower.

The surface-treated zinc oxide particles after the heat treatment can be cracked, for example, using a known pulverizer. Examples of the pulverizer include atomizers, hammer mills, jet mills, impeller mills, and pin mills.

With the method for producing surface-treated zinc oxide particles according to the present embodiment, since the surface treatment is performed in which the zinc oxide particles having a BET specific surface area of 1.5 m²/g or higher and 8 m²/g or lower is mixed with the alkylalkoxysilane having an alkyl group having 6 or more and 10 or less carbon atoms in an amount of 1.2 parts by mass or more and 3.5 parts by mass with respect to 100 parts by mass of the zinc oxide particles and the content of the solvent is 2% by mass or less with respect to the total mass of the zinc oxide particles and the surface treatment agent, surface-treated zinc oxide particles with suppressed agglomeration can be obtained.

### [Dispersion liquid]

The dispersion liquid according to the present embodiment contains the surface-treated zinc oxide particles according to the present embodiment, and a dispersion medium.

The dispersion liquid according to the present embodiment also includes a high-viscosity paste-like dispersion.

The dispersion medium is not particularly limited as long as it can be formulated in a cosmetic material and can disperse the surface-treated zinc oxide particles.

As the dispersion medium, for example, water, alcohols, esters, ethers, natural oils, ester oils, silicone oils, and the like are suitably used.

Examples of the alcohols include methanol, ethanol, n-propanol, isopropanol, n-butanol, 2-butanol, octanol, and glycerin.

Examples of the esters include ethyl acetate, butyl acetate, ethyl lactate, propylene glycol monomethyl ether acetate, propylene glycol monoethyl ether acetate, and g-butyrolactone.

Examples of the ethers include diethyl ether, ethylene glycol monomethyl ether (methyl cellosolve), ethylene glycol monoethyl ether (ethyl cellosolve), ethylene glycol monobutyl ether (butyl cellosolve), diethylene glycol monomethyl ether, and diethylene glycol monoethyl ether.

In addition, other dispersion media such as ketones, aromatic hydrocarbons, cyclic hydrocarbons, amides, chain-like polysiloxanes, cyclic polysiloxanes, modified polysiloxanes, hydrocarbon oils, ester oils, silicone oils, higher fatty acids, and higher alcohols are used.

Examples of the ketones include acetone, methyl ethyl ketone, methyl isobutyl ketone, acetyl acetone, and cyclohexanone.

Examples of the aromatic hydrocarbons include benzene, toluene, xylene, and ethyl benzene.

Examples of the cyclic hydrocarbons include cyclohexane and the like.

Examples of the amides include dimethylformamide, N,N-dimethylacetoacetamide, and N-methylpyrrolidone.

Examples of the chain-like polysiloxanes include dimethyl polysiloxane, methyl phenyl polysiloxane, and diphenyl polysiloxane.

Examples of the cyclic polysiloxanes include octamethylcyclotetrasiloxane, decamethylcyclopentasiloxane, and dodecamethylcyclohexasiloxane. Examples of the modified polysiloxanes include amino-modified polysiloxane, polyether-modified polysiloxane, alkyl-modified polysiloxane, and fluorine-modified polysiloxane.

Examples of the hydrocarbon oil include liquid paraffin, squalane, isoparaffin, branched chain-like light paraffin, petrolatum, and ceresin.

Examples of the ester oil include isopropyl myristate, cetyl isooctanoate, and glyceryl trioctanoate.

Examples of the silicone oil include decamethylcyclopentasiloxane, dimethylpolysiloxane, and methylphenylpolysiloxane.

Examples of the higher fatty acid include lauric acid, myristic acid, palmitic acid, and stearic acid.

Examples of the higher alcohol include lauryl alcohol, cetyl alcohol, stearyl alcohol, hexyl dodecanol, and isostearyl alcohol.

The above-described dispersion medium may be used alone or in combination with two or more types thereof.

The dispersion liquid according to the present embodiment may contain a commonly used additive as long as the characteristics thereof are not impaired.

As the additive, for example, a preservative, a dispersant, a dispersion aid, a stabilizer, a water-soluble binder, a viscosity improver, an oil-soluble chemical, an oil-soluble coloring agent, oil-soluble proteins, a UV absorber, or the like is suitably used.

In the dispersion liquid according to the present embodiment, a particle diameter (D50) of the surface-treated zinc oxide particles in a case where a cumulative volume percentage in a particle size distribution is 50% can be optionally selected, but it is preferably 600 nm or lower, more preferably 500 nm or lower, and still more preferably 400 nm or lower.

The lower limit value of the D50 is not particularly limited, and may be, for example, 130 nm or higher, 140 nm or higher, or 150 nm or higher. The upper limit value and lower limit value of the D50 can be combined randomly.

In addition, in the dispersion liquid according to the present embodiment, a particle diameter (D90) of the surface-treated zinc oxide particles in a case where a cumulative volume percentage in a particle size distribution is 90% can be optionally selected, but it is preferably 1 µm or lower, more preferably 900 nm or lower, and still more preferably 800 nm or lower.

The lower limit value of the D90 is not particularly limited, and may be, for example, 150 nm or higher, 200 nm or higher, or 250 nm or higher. The upper limit value and lower limit value of the D90 can be combined randomly.

In a case where the D50 of the dispersion liquid is 600 nm or lower, when a cosmetic material produced using this dispersion liquid is applied to the skin, it is easy to uniformly distribute the surface-treated zinc oxide particles and to improve an ultraviolet shielding effect, which is preferable. In addition, in a case where the D90 of the dispersion liquid is 1 µm or lower, the transparency of the dispersion liquid is high, and transparency of a cosmetic material produced using this dispersion liquid is also high, which is preferable.

That is, in a case where the D50 and the D90 of the dispersion liquid according to the present embodiment are within the above-described ranges, it is possible to obtain a dispersion liquid having excellent transparency and excellent ultraviolet shielding properties. In addition, the cosmetic material produced using the dispersion liquid are also excellent in terms of transparency and ultraviolet shielding properties.

The cumulative volume percentage in the particle size distribution of the dispersion liquid can be measured using a dynamic light scattering particle size distribution analyzer.

The content of the surface-treated zinc oxide particles in the dispersion liquid according to the present embodiment can be appropriately adjusted according to the desired characteristics.

In a case where the dispersion liquid according to the present embodiment is used in a cosmetic material, the content of the surface-treated zinc oxide particles in the dispersion liquid can be optionally selected, but is preferably 10% by mass or more, more preferably 20% by mass or more, and still more preferably 30% by mass or more. In addition, the content of the surface-treated zinc oxide particles in the dispersion liquid is preferably 90% by mass or less, more preferably 85% by mass or less, and still more preferably 80% by mass or less. The upper limit value and the lower limit value of the content of the surface-treated zinc oxide particles in the dispersion liquid can be combined randomly.

In a case where the content of the surface-treated zinc oxide particles in the dispersion liquid is within the above-described range, the surface-treated zinc oxide particles are contained at a high concentration. Therefore, a degree of freedom in formulation can be improved, and a viscosity of the dispersion liquid can be adjusted to a level which makes it easy to handle.

The viscosity of the dispersion liquid according to the present embodiment can be optionally selected, but is preferably 5 Pa·s or higher, more preferably 8 Pa·s or higher, still more preferably 10 Pa·s or higher, and most preferably 15 Pa·s or higher. In addition, the viscosity of the dispersion liquid is preferably 300 Pa·s or lower, more preferably 100 Pa·s or lower, still more preferably 80 Pa·s or lower, and most preferably 60 Pa·s or lower. The upper limit value and lower limit value of the viscosity of the dispersion liquid can be combined randomly.

In a case where the viscosity of the dispersion liquid is within the above-described range, it is possible to obtain a dispersion liquid which is easy to handle even in a case where the dispersion liquid contains a high concentration of solid content (surface-treated zinc oxide particles).

A method for producing the dispersion liquid according to the present embodiment is not particularly limited. Examples thereof include a method in which the surface-treated zinc oxide particles according to the present embodiment and the dispersion medium are mechanically mixed with each other using a known dispersion device.

A dispersion device can be optionally selected, and examples thereof include a stirrer, a planetary mixer, a homomixer, an ultrasonic homogenizer, a sand mill, a ball mill, and a roll mill.

The dispersion liquid according to the present embodiment can be used for, in addition to the cosmetic material, paints and the like, having an ultraviolet shielding function, a gas transmission-suppressing function, or the like.

With the dispersion liquid according to the present embodiment, since the dispersion liquid contains the surface-treated zinc oxide particles according to the present embodiment, when blended into a cosmetic material, the feeling of roughness is suppressed, and transparency and ultraviolet shielding properties are excellent.

### [Composition]

The composition according to the present embodiment contains the surface-treated zinc oxide particles according to the present embodiment, a resin, and a dispersion medium.

A content of the surface-treated zinc oxide particles in the composition according to the present embodiment can be appropriately adjusted according to the desired characteristics. The above-described content is preferably, for example, 10% by mass or more and 40% by mass or less, and more preferably 20% by mass or more and 30% by mass or less.

In a case where the content of the surface-treated zinc oxide particles in the composition is within the above-described range, the high concentration of solid content (surface-treated zinc oxide particles) allows for the sufficient manifestation of the characteristics of the surface-treated zinc oxide particles, and a composition in which the surface-treated zinc oxide particles are uniformly dispersed can be obtained.

The resin is not particularly limited as long as it is generally used for industrial applications, and examples thereof include an acrylic resin, an epoxy resin, a urethane resin, a polyester resin, and a silicone resin.

A content of the resin in the composition according to the present embodiment is not particularly limited, and is appropriately adjusted depending on the intended characteristics of the composition.

The dispersion medium is not particularly limited as long as it is generally used for industrial applications, and examples thereof include water, alcohols such as methanol, ethanol, and propanol, methyl acetate, ethyl acetate, toluene, methyl ethyl ketone, and methyl isobutyl ketone.

A content of the dispersion medium in the composition according to the present embodiment is not particularly limited, and is appropriately adjusted depending on the intended characteristics of the composition.

The composition according to the present embodiment may contain a commonly used additive as long as the characteristics thereof are not impaired.

Examples of the additive include a polymerization initiator, a dispersant, and a preservative.

A method for producing the composition according to the present embodiment is not particularly limited, and examples thereof include a method in which the surface-treated zinc oxide particles according to the present embodiment, the resin, and the dispersion medium are mechanically mixed with each other using a known dispersion device.

In addition, examples thereof include a method in which the above-described dispersion liquid and the resin are mechanically mixed with each other using a known mixing device.

Examples of the mixing device include a stirrer, a planetary mixer, a homogenizer, and an ultrasonic homogenizer.

A coating film can be formed by applying the composition according to the present embodiment to an optional base material, for example, a plastic base material such as a polyester film by a normal application method such as a roll coating method, a flow coating method, a spray coating method, a screen printing method, a brush coating method, and an immersion method. These coating films can be used for various applications, such as an ultraviolet shielding film and a gas barrier film.

With the composition according to the present embodiment, since the composition contains the surface-treated zinc oxide particles according to the present embodiment, the surface-treated zinc oxide particles can be easily mixed with the resin, and the composition exhibits excellent transparency and ultraviolet shielding properties.

### [Cosmetic material]

The cosmetic material according to one embodiment of the present invention contains at least one selected from the group consisting of the surface-treated zinc oxide particles according to the present embodiment and the dispersion liquid according to the present embodiment. Alternatively, the cosmetic material according to one embodiment of the present invention contains at least one selected from the group consisting of the surface-treated zinc oxide particles according to the present embodiment, the dispersion liquid according to the present embodiment, and the composition according to the present embodiment.

The cosmetic material according to another embodiment contains a cosmetic base raw material and at least one selected from the group consisting of the surface-treated zinc oxide particles according to the present embodiment and the dispersion liquid according to the present embodiment. Alternatively, the cosmetic material according to another embodiment contains a cosmetic base raw material, and at least one selected from the group consisting of the surface-treated zinc oxide particles according to the present embodiment, the dispersion liquid according to the present embodiment, and the composition according to the present embodiment.

The cosmetic base raw material refers to various raw materials which form a main body of a cosmetic product. Examples of the cosmetic base raw material include an oil-based raw material, a water-based raw material, a surfactant, and a particulate raw material.

The oil-based raw material can be optionally selected, and examples thereof include oils and fats, higher fatty acids, higher alcohols, and ester oils.

The water-based raw material can be optionally selected, and examples thereof include purified water, alcohol, and a thickener.

The powder raw material can be optionally selected, and examples thereof include a colored pigment, a white pigment, a pearlescent agent, and an extender pigment.

The cosmetic material according to the present embodiment can be obtained by, for example, blending the dispersion liquid according to the present embodiment into a cosmetic base raw material in the related art, such as an emulsion, a cream, a foundation, a lip stick, a blush, and an eye shadow.

The cosmetic material according to the present embodiment is obtained, for example, by blending the surface-treated zinc oxide particles according to the present embodiment into an oil phase or a water phase to prepare an O/W type or W/O type emulsion, and then blending the emulsion with the cosmetic base raw material.

A content of the surface-treated zinc oxide particles in the cosmetic material according to the present embodiment can be appropriately adjusted according to the desired characteristics. For example, the lower limit of the content of the surface-treated zinc oxide particles described above may be 0.01% by mass or more, 0.1% by mass or more, or 1% by mass or more. In addition, the upper limit of the content of the surface-treated zinc oxide particles may be 50% by mass or less, 40% by mass or less, or 30% by mass or less. The upper limit value and the lower limit value of the content of the surface-treated zinc oxide particles in the cosmetic material can be combined randomly.

Hereinafter, a sunscreen cosmetic material will be specifically described.

In the sunscreen cosmetic material, in order to effectively shield ultraviolet rays, particularly long-wavelength ultraviolet rays (UVA), and in order to achieve a favorable feeling of use with minimal powderiness and creaking, it is also preferable to adjust the content of the surface-treated zinc oxide particles. For example, the lower limit of the content of the surface-treated zinc oxide particles in the sunscreen cosmetic material is preferably 0.01% by mass or more, more preferably 0.1% by mass or more, and still more preferably 1% by mass or more. In addition, the upper limit of the content of the surface-treated zinc oxide particles in the sunscreen cosmetic material preparation may be 50% by mass or less, 40% by mass or less, or 30% by mass or less. The upper limit value and the lower limit value of the content of the surface-treated zinc oxide particles in the sunscreen cosmetic material can be combined randomly. In addition, within the above-described range, a preferred range can be optionally selected, such as 5% by mass to 15% by mass or 10% by mass to 20% by mass.

The sunscreen cosmetic material may contain inorganic fine particles or inorganic pigment other than the surface-treated zinc oxide particles, a hydrophilic dispersion medium, a hydrophobic dispersion medium, fats and oils, a surfactant, a moisturizer, a viscosity improver, a pH adjuster, a nutrient, an antioxidant, a fragrance, a preservative, a dispersant, an antifoaming agent, a coloring agent, a cosmetic ingredient, a polymeric substance, a bio-derived ingredient, a plant-derived ingredient, an antibacterial agent, a bactericide, a fungicide, an aqueous Ingredient, an oily ingredient, a vitamin supplement, an emulsifier, a stabilizer, a solubilizer, a pearlescent agent, a refatting substance, or the like as necessary.

Examples of the hydrophobic dispersion medium include hydrocarbon oils, ester oils, silicone oils, higher fatty acids, and higher alcohols.

Examples of the hydrocarbon oil include liquid paraffin, squalane, isoparaffin, branched chain-like light paraffin, petrolatum, and ceresin.

Examples of the ester oil include isopropyl myristate, cetyl isooctanoate, and glyceryl trioctanoate.

Examples of the silicone oil include decamethylcyclopentasiloxane, dimethylpolysiloxane, and methylphenylpolysiloxane.

Examples of the higher fatty acid include lauric acid, myristic acid, palmitic acid, and stearic acid.

Examples of the higher alcohol include lauryl alcohol, cetyl alcohol, stearyl alcohol, hexyl dodecanol, and isostearyl alcohol.

Examples of the inorganic fine particles or inorganic pigment other than the surface-treated zinc oxide particles contained in the cosmetic material include calcium carbonate, calcium phosphate (apatite), magnesium carbonate, calcium silicate, magnesium silicate, aluminum silicate, kaolin, talc, titanium oxide, aluminum oxide, yellow oxide of iron, γ-iron oxide, cobalt titanate, cobalt violet, and silicon oxide.

The sunscreen cosmetic material may further contain at least one organic ultraviolet absorber. The cosmetic material containing both the surface-treated zinc oxide particles and the organic ultraviolet absorber is preferable because the cosmetic material broadens the ultraviolet shielding region through a booster effect and also enhances ultraviolet shielding properties.

Examples of the organic ultraviolet absorber include a benzotriazole-based ultraviolet absorber, a benzoyl methane-based ultraviolet absorber, a benzoic acid-based ultraviolet absorber, an anthranilic acid-based ultraviolet absorber, a salicylic acid-based ultraviolet absorber, a cinnamic acid-based ultraviolet absorber, a silicone-based cinnamic acid ultraviolet absorber, and a triazine-based ultraviolet absorber.

Examples of the benzotriazole-based ultraviolet absorber include 2,2'-hydroxy-5-methylphenylbenzotriazole, 2-(2'-hydroxy-5'-t-octylphenyl)benzotriazole, and 2-(2'-hydroxy-5'-methylphenyl)benzotriazole.

Examples of the benzoyl methane-based ultraviolet absorber include dibenzalazine, dianisoylmethane, 4-tert-butyl-4'-methoxydibenzoylmethane, 1-(4'-isopropylphenyl)-3-phenyl propane-1,3-dione, and 5-(3,3'-dimethyl-2-norbornylidene)-3-pentane-2-one.

Examples of the benzoic acid-based ultraviolet absorber include para-aminobenzoic acid (PABA), PABA monoglycerin ester, N,N-dipropoxy PABA ethyl ester, N,N-diethoxy PABA ethyl ester, N,N-dimethyl PABA ethyl ester, N,N-dimethyl PABA butyl ester, and N,N-dimethyl PABA methyl ester.

Examples of the anthranilic acid-based ultraviolet absorber include homomenthyl-N-acetyl anthranilate and the like.

Examples of the salicylic acid-based ultraviolet absorber include amyl salicylate, menthyl salicylate, homo menthyl salicylate, octyl salicylate, phenyl salicylate, benzyl salicylate, and p-2-propanol phenyl salicylate.

Examples of the cinnamic acid-based ultraviolet absorber include octyl methoxycinnamate(ethylhexyl methoxycinnamate), di-para methoxy cinnamate-mono-2-glyceryl ethylhexanoate, octyl cinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxy cinnamate, isopropyl-p-methoxy cinnamate, isoamyl-p-methoxy cinnamate, octyl-p-methoxy cinnamate(2-ethylhexyl-p-methoxy cinnamate), 2-ethoxyethyl-p-methoxy cinnamate, cyclohexyl-p-methoxy cinnamate, ethyl-α-cyano-β-phenyl cinnamate, 2-ethylhexyl-α-cyano-β-phenyl cinnamate, and glyceryl mono-2-ethylhexanoyl-diparamethoxy cinnamate.

Examples of the silicone-based cinnamic acid ultraviolet absorber include [3-bis(trimethylsiloxy)methylsilyl-1-methylpropyl]-3,4,5-trimethoxy cinnamate, [3-bis(trimethylsiloxy)methylsilyl-3-methylpropyl]-3,4,5-trimethoxy cinnamate, [3-bis(trimethylsiloxy)methylsilylpropyl]-3,4,5-trimethoxy cinnamate, [3-bis(trimethylsiloxy)methylsilylbutyl]-3,4,5-trimethoxy cinnamate, [3-tris(trimethylsiloxy)silylbutyl]-3,4,5-trimethoxy cinnamate, and [3-tris(trimethylsiloxy)silyl-1-methylpropyl]-3,4-dimethoxy cinnamate.

Examples of the triazine-based ultraviolet absorber include bis-ethylhexyloxyphenol methoxyphenyl triazine, ethylhexyl triazone, methylene bis-benzotriazolyl tetramethylbutylphenol, tris-biphenyl triazine, and diethylhexyl butamido triazone.

Examples of the organic ultraviolet absorber other than the above-described ultraviolet absorbers include 3-(4'-methylbenzylidene)-d,l-camphor, 3-benzylidene-d,l-camphor, urocanic acid, ethyl urocanate esters, 2-phenyl-5-methylbenzoxane, 5-(3,3'-dimethyl-2-norbornylidene)-3-pentane-2-one, silicone-modified ultraviolet absorbers, and fluorine-modified ultraviolet absorbers. The above-described ultraviolet absorber may be used alone or in combination with two or more types thereof.

A critical wavelength of the cosmetic material according to the present embodiment is preferably 370 nm or higher. In a case where the critical wavelength of the cosmetic material is 370 nm or higher, a wide range of long-wavelength ultraviolet rays (UVA) and short-wavelength ultraviolet rays (UVB) can be shielded.

The cosmetic material according to the present embodiment contains at least one selected from the group consisting of the surface-treated zinc oxide particles according to the present embodiment, the dispersion liquid according to the present embodiment, and the composition according to the present embodiment. Therefore, it is possible to obtain a cosmetic material with excellent transparency and ultraviolet shielding properties, and a favorable feeling of use, in which the feeling of roughness of the surface-treated zinc oxide particles is suppressed.

### Examples

Hereinafter, the present invention will be more specifically described with Examples and Comparative Examples, but the present invention is not limited to Examples.

### [Example 1]

### "Production of surface-treated zinc oxide particles"

100 parts by mass of zinc oxide particles A1 (BET specific surface area: 5 m²/g, manufactured by SUMITOMO OSAKA CEMENT Co., Ltd.), 1.5 parts by mass of octyltriethoxysilane (trade name: Silquest A-137, manufactured by Momentive Performance Materials) were mixed in a Henschel mixer heated to 60°C at a circumferential speed of 15 m/s.

Next, the mixture was heat-treated at 95°C for 6 hours under reduced pressure.

Next, the obtained dried product was cracked using a hammer mill at 16,000 rpm to obtain surface-treated zinc oxide particles B1 of Example 1.

### (Content of octyltriethoxysilane)

A drying loss of 2 g of the surface-treated zinc oxide particles of Example 1 at 105°C for 2 hours and an ignition loss of 2 g of the surface-treated zinc oxide particles of Example 1 at 500°C for 4 hours were measured, and a content of octyltriethoxysilane was calculated using the above-described general formulae (1) to (3). The result is shown in Table 1.

### (Content of Si)

A content of Si in the surface-treated zinc oxide particles of Example 1 was measured using an ICP emission spectrometry apparatus (trade name: ICP-AES 700-ES, manufactured by Varian, Inc.) by the following method.

0.2 g of the surface-treated zinc oxide particles of Example 1 was placed into a platinum crucible, and the temperature was gradually raised to 700°C in an electric furnace to ash the surface-treated zinc oxide particles. 2 g of lithium tetraborate was added to the ashed sample, and the sample was heated in the electric furnace to 925°C to be molten.

Next, the molten sample was placed into a 100 mL tall beaker along with the platinum crucible.

Next, 70 mL of warm water and 8 mL of nitric acid were added to the tall beaker, and the mixture was heated and stirred with a hot stirrer to dissolve the sample. The dissolved solution was transferred to a 200 mL volumetric flask and brought to volume, and this solution was used as a sample solution. A Y standard solution was added to the sample solution so that the concentration of Y was to be 1,000 ppm to obtain a sample solution for measurement. The sample solution was measured using the ICP emission spectrometry apparatus, and the content of Si in the surface-treated zinc oxide particles was quantified by a calibration curve method. The calibration curve was created using a solution obtained by adding Y as an internal standard substance, the lithium tetraborate, and the nitric acid to the element standard solution with a known concentration, ensuring the same concentration as the sample solution.

### (Measurement of particle size distribution)

A dry dispersion unit (trade name: AeroS, manufactured by Malvern Panalytical Ltd.) was mounted on a laser diffraction particle diameter measuring device (trade name: Mastersizer 3000, manufactured by Malvern Panalytical Ltd.), and a volume-based particle size distribution of the surface-treated zinc oxide particles of Example 1 was measured in a dry manner. The result is shown in Table 1.

### (Measurement of hydroxyl group treatment rate)

### "Production of red coloring agent"

1 mmol of 2,2'-dihydroxyazobenzene, 1 mmol of diphenyltin(IV) oxide, and 30 mL of acetone were mixed with each other to prepare a mixed solution.

Next, the mixed solution was stirred at 70°C for 3 hours to perform a dehydration reaction, coordinating the diphenyltin oxide with the 2,2'-dihydroxyazobenzene.

The mixed solution after the dehydration reaction was filtered, the filtrate was collected, and the solvent was distilled off from the filtrate to obtain the red coloring agent represented by General Formula (4) described above.

### "Preparation of solution for evaluation"

250 nmol (0.12 mg) of the obtained red coloring agent was dissolved in toluene to prepare 5 mL of a solution C1 for evaluation, having a concentration of 5 × 10⁻⁵ mol/L. An absorbance C2 of the solution C1 for evaluation at 545 nm was measured.

The zinc oxide particles A1 were added to the solution C1 in an amount of 4.0 mg, and the mixture was stirred and mixed at 60°C for 4 hours to adjust a mixed solution. The mixed solution was filtered through a syringe filter (0.2 µm), and an absorbance A2 of the filtrate at 545 nm was measured.

4.0 mg of the surface-treated zinc oxide particles B1 of Example 1 was added to the solution C1, and the mixture was stirred and mixed at 60°C for 4 hours to adjust a mixed solution. The mixed solution was filtered through a syringe filter (0.2 µm), and an absorbance B2 of the filtrate at 545 nm was measured.

The amounts of the red coloring agent adsorbed on the zinc oxide particles and the surface-treated zinc oxide particles were each calculated using the above-described formulae (5) and (6). Amount B3 of coloring agent adsorbed on surface-treated zinc oxide particles = ((B2 - C2)/C2) × 250 × 10-9 (mol) /4 × 10-3 (g) Amount A3 of coloring agent adsorbed on zinc oxide particles = ((A2 - C2) /C2) × 250 × 10-9 (mol)/4 × 10-3 (g)

A hydroxyl group treatment rate of Example 1 was calculated using the above-described formula (7). The result is shown in Table 1. Hydroxyl group treatment rate = 100 - (B3/A3 × 100)

### (Hydrophobicity evaluation by critical ethanol method)

The critical ethanol method is a method of adding a sample to a solution obtained by mixing water and ethanol, and then observing whether the sample precipitates. In addition, the critical ethanol method is a method which evaluates hydrophobicity of surfaces of zinc oxide particles by increasing an ethanol ratio in a case where the sample does not precipitate and by increasing a water ratio in a case where the sample does precipitate, determining the ethanol ratio required for the sample to precipitate. As the ethanol ratio increases, the surfaces of the zinc oxide particles are more hydrophobic, indicating a higher metal hydroxyl group treatment rate.

The surface-treated zinc oxide particles of Example 1 were evaluated by the critical ethanol method. As a result, it was found that 10 or more particles were precipitated when the ethanol ratio was 40%. Table 1 shows the ethanol concentration at which 10 or more surface-treated zinc oxide particles were precipitated.

### (Evaluation of b*)

b* of the surface-treated zinc oxide particles of Example 1 was measured using a spectrophotometer (trade name: Spectro Color Meter SE7700, manufactured by Tokyo Denshoku CO., LTD.). The measurement was performed under reflection (2-degree visual field) measurement conditions with a measurement diameter of 10 mm, using a D65 light source.

As a measurement sample, 10 g of the surface-treated zinc oxide particles of Example 1 was put into a 30 mL screw tube and tapped 30 times on a table, and the bottom surface of this screw tube was used as a measurement surface. The result is shown in Table 1.

### (Evaluation of feeling of use (texture))

Texture of the surface-treated zinc oxide particles of Example 1 was evaluated as follows. The surface-treated zinc oxide particles were taken between the thumb and index finger, and the presence or absence of feeling of roughness when rubbed together was evaluated. A case where the roughness was not felt was evaluated as "○" (good), a case where the roughness was slightly felt was evaluated as "△" (acceptable), and a case where the roughness was felt was evaluated as "×" (unacceptable). The result is shown in Table 1.

As the feeling of roughness of the surface-treated zinc oxide particles was smaller, the texture was better, with "○" indicating the best texture, followed by "△" and then "×".

### [Example 2]

Surface-treated zinc oxide particles of Example 2 were obtained in the same manner as in Example 1, except that 100 parts by mass of zinc oxide particles and 2.0 parts by mass of octyltriethoxysilane were used.

The content of the surface treatment agent, the particle size distribution, the hydroxyl group treatment rate, the hydrophobicity, b*, and the feeling of use were measured in the same manner as in Example 1. The result is shown in Table 1.

### [Example 3]

Surface-treated zinc oxide particles of Example 3 were obtained in the same manner as in Example 1, except that 100 parts by mass of zinc oxide particles and 3.0 parts by mass of octyltriethoxysilane were used.

The content of the surface treatment agent, the particle size distribution, the hydroxyl group treatment rate, the hydrophobicity, b*, and the feeling of use were measured in the same manner as in Example 1. The result is shown in Table 1.

### [Comparative Example 1]

Surface-treated zinc oxide particles of Comparative Example 1 were obtained in the same manner as in Example 1, except that 100 parts by mass of zinc oxide particles and 0.5 parts by mass of octyltriethoxysilane were used.

The content of the surface treatment agent, the particle size distribution, the hydroxyl group treatment rate, the hydrophobicity, b*, and the feeling of use were measured in the same manner as in Example 1. The result is shown in Table 1.

### [Comparative Example 2]

Surface-treated zinc oxide particles of Comparative Example 2 were obtained in the same manner as in Example 1, except that 100 parts by mass of zinc oxide particles and 1.0 part by mass of octyltriethoxysilane were used.

The content of the surface treatment agent, the particle size distribution, the hydroxyl group treatment rate, the hydrophobicity, b*, and the feeling of use were measured in the same manner as in Example 1. The result is shown in Table 1.

### [Comparative Example 3]

Surface-treated zinc oxide particles of Comparative Example 3 were obtained in the same manner as in Example 1, except that 100 parts by mass of zinc oxide particles and 4.0 parts by mass of octyltriethoxysilane were used.

The content of the surface treatment agent, the particle size distribution, the hydroxyl group treatment rate, the hydrophobicity, b*, and the feeling of use were measured in the same manner as in Example 1. The result is shown in Table 1.

### [Comparative Example 4]

Surface-treated zinc oxide particles of Comparative Example 4 were obtained in the same manner as in Example 1, except that 100 parts by mass of zinc oxide particles and 5.0 parts by mass of octyltriethoxysilane were used.

The content of the surface treatment agent, the particle size distribution, the hydroxyl group treatment rate, the hydrophobicity, b*, and the feeling of use were measured in the same manner as in Example 1. The result is shown in Table 1.

### [Comparative Example 5]

Surface-treated zinc oxide particles of Comparative Example 5 were obtained in the same manner as in Example 1, except that 100 parts by mass of zinc oxide particles, 2.0 parts by mass of octyltriethoxysilane, and 36.0 parts by mass of isopropyl alcohol were used.

The content of the surface treatment agent, the particle size distribution, the hydroxyl group treatment rate, the hydrophobicity, b*, and the feeling of use were measured in the same manner as in Example 1. The result is shown in Table 1.

**[Table 1]**

| | | Example 1 | Example 2 | Example 3 | Comparative Example 1 | Comparative Example 2 | Comparative Example 3 | Comparative Example 4 | Comparative Example 5 |
|---|---|---|---|---|---|---|---|---|---|
| Production conditions | Specific surface area [m²/g] | 5 | 5 | 5 | 5 | 5 | 5 | 5 | 5 |
| | Zinc oxide [part by mass] | 100 | 100 | 100 | 100 | 100 | 100 | 100 | 100 |
| | Octyltriethoxysilane [part by mass] | 1.5 | 2.0 | 3.0 | 0.5 | 1.0 | 4.0 | 5.0 | 2.0 |
| | Isopropyl alcohol [part by mass] | 0.0 | 0.0 | 0.0 | 0.0 | 0.0 | 0.0 | 0.0 | 36.0 |
| | Drying loss [% by mass] | 0.07 | 0.08 | 0.05 | 0.07 | 0.09 | 0.07 | 0.08 | 0.10 |
| | Ignition loss [% by mass] | 0.37 | 0.40 | 0.42 | 0.27 | 0.37 | 0.45 | 0.47 | 0.39 |
| | Content of surface treatment agent [% by mass] | 0.73 | 0.78 | 0.90 | 0.49 | 0.68 | 0.93 | 0.95 | 0.71 |
| Evaluation results | Amount of Si [%] | 0.07 | 0.10 | 0.20 | 0.03 | 0.05 | 0.35 | 0.54 | 0.09 |
| | Hydroxyl group treatment rate [%] | 100.0 | 100.0 | 99.0 | 51.4 | 96.5 | 99.4 | 99.5 | 100.0 |
| | Hydrophobicity evaluation [%] | 40 | 42 | 40 | 20 | 38 | 40 | 40 | 36 |
| | D98 [µm] | 34.7 | 32.6 | 34.4 | 42.3 | 38.0 | 41.5 | 49.9 | 48.1 |
| | b* | 6.1 | 6.2 | 6.1 | 4.7 | 5.8 | 6.6 | 6.4 | 5.7 |
| | Texture | ○ | ○ | ○ | × | × | × | × | × |

By comparing Examples 1 to 3 with Comparative Examples 1 to 5, it was found that the surface-modified zinc oxide particles produced using the zinc oxide particles having a BET specific surface area of 1.5 m²/g or higher and 8 m²/g or lower and the octyltriethoxysilane with a mass ratio in a range of 100:1.2 to 100:3.5 had high hydrophobicity, suppressed feeling of roughness, and small b*.

### Industrial Applicability

The present invention provides surface-treated zinc oxide particles with a suppressed feeling of roughness, a dispersion liquid and a cosmetic material containing the surface-treated zinc oxide particles, and a method for producing the surface-treated zinc oxide particles. The surface-treated zinc oxide particles of the present invention exhibit excellent color and feeling of use. Therefore, the surface-treated zinc oxide particles of the present invention are easy to ensure designed quality when applied to dispersion liquids, compositions, paints, and cosmetic materials, and have high industrial value.

## Claims

1. Surface-treated zinc oxide particles which are zinc oxide particles surface-treated with a surface treatment agent,
wherein a BET specific surface area of the zinc oxide particles is 1.5 m²/g or higher and 8 m²/g or lower, the surface treatment agent is an alkylalkoxysilane having an alkyl group having 6 or more and 10 or less carbon atoms,
a content of the surface treatment agent is 0.70% by mass or more and 0.92% by mass or less, and
a particle diameter D98 in a case where a cumulative volume percentage in a dry particle size distribution is 98% is 40 µm or lower.

2. The surface-treated zinc oxide particles according to Claim 1,
wherein the surface treatment agent is at least one of octyltrimethoxysilane or octyltriethoxysilane.

3. A dispersion liquid comprising:
the surface-treated zinc oxide particles according to Claim 1 or 2; and
a dispersion medium.

4. A cosmetic material comprising:
at least one selected from the group consisting of the surface-treated zinc oxide particles according to Claim 1 or 2 or the dispersion liquid according to Claim 3.

5. A method for producing surface-treated zinc oxide particles, which is a method for producing the surface-treated zinc oxide particles according to Claim 1 or 2, the method comprising:
a step of subjecting zinc oxide particles to a surface treatment by mixing the zinc oxide particles with a surface treatment agent;
a step of subjecting the surface-treated zinc oxide particles to a heat treatment; and
a step of cracking the surface-treated zinc oxide particles after the heat treatment,
wherein a BET specific surface area of the zinc oxide particles is 1.5 m²/g or higher and 8 m²/g or lower, the surface treatment agent is an alkylalkoxysilane having an alkyl group having 6 or more and 10 or less carbon atoms,
a mixing amount of the surface treatment agent is 1.2 parts by mass or more and 3.5 parts by mass or less with respect to 100 parts by mass of the zinc oxide particles, and
in the step of subjecting the zinc oxide particles to the surface treatment, a content of a solvent is 2% by mass or less with respect to a total mass of the zinc oxide particles and the surface treatment agent.
